Europäisches Patentamt

European Patent Office

Office européen des brevets

(11) Publication number: **0 394 928**
**A1**

(12) **EUROPEAN PATENT APPLICATION**

(21) Application number: **90107671.1**

(22) Date of filing: **23.04.90**

(51) Int. Cl.⁵: **A61K 31/765,** //(A61K31/765, 31:52)

(30) Priority: **24.04.89 US 342939**

(43) Date of publication of application:
**31.10.90 Bulletin 90/44**

(84) Designated Contracting States:
**AT BE CH DE DK ES FR GB GR IT LI LU NL SE**

(71) Applicant: **SYNTEX (U.S.A.) INC.**
**3401 Hillview Avenue**
**Palo Alto California 94303(US)**

(72) Inventor: **Verheyden, Julien P.H.**
**244 Marvin Avenue**
**Los Altos, California 94022(US)**
Inventor: **Wong, Albert Bakwei**
**774 Pacheco Drive**
**Milpitas, California 95035(US)**

(74) Representative: **Barz, Peter, Dr. et al**
**Patentanwälte Dipl.-Ing. G. Dannenberg Dr.**
**P. Weinhold, Dr. D. Gudel Dipl.-Ing. S.**
**Schubert, Dr. P. Barz Siegfriedstrasse 8**
**D-8000 München 40(DE)**

(54) **Topical antiviral pharmaceutical composition containing a nonionic surfactant.**

(57) A topical pharmaceutical composition useful in treating viral infections in mammals includes a therapeutically effective amount of an antiviral active agent, a pharmaceutically acceptable nonionic surfactant in quantity sufficient to enhance the antiviral activity of said composition, and optionally, pharmaceutically acceptable excipients in quantity sufficient to make weight.

A method is disclosed for treating viral infections in mammals by topically applying the aforesaid pharmaceutical composition in quantity sufficient to cover the site of the infection and the areas adjacent.

A method is also disclosed for making the aforesaid composition.

EP 0 394 928 A1

## TOPICAL ANTIVIRAL PHARMACEUTICAL COMPOSITION CONTAINING A NONIONIC SURFACTANT

This invention relates to topical antiviral compositions, a method of treating viral infections in mammals and a method of manufacture of aforesaid composition; particularly to topical antiviral compositions having an amount of a pharmaceutically acceptable surfactant sufficient to enhance antiviral activity; specifically to a topical antiviral composition which includes an effective amount of 9-[1,3-di-(1-adamantylcarboxy)-2-propoxymethyl]guanine in combination with an amount of a pharmaceutically acceptable nonionic surfactant sufficient to enhance antiviral activity; more specifically to a topical antiviral composition which includes an effective amount of 9-(2-hydroxyethoxymethyl)guanine in combination with an amount of a pharmaceutically acceptable nonionic surfactant sufficient to enhance antiviral activity.

Herpes simplex viruses (HSVs) are among the most common and persistent infectious agents known to man. Infections by herpes type virus create significant health and social problems. The infections are transmitted from an infected subject by close or intimate bodily contact. An infected subject may also auto-inoculate, that is, spread the viral infection from one part of the body to another. The infections are especially troublesome because of their persistent nature and ease of transmission. The problem is further exacerbated by the fact that there is no effective vaccine available and, once infected, there is no way to cure the subject of the infection.

Lesions appear after a primary infection or after a secondary infection due to resurgence of the virus from the latency in the neural ganglions. Lesions are formed during a stage of the infection where there is an increase in the rate of viral multiplication. This sudden increase causes the death of significant numbers of cells resulting in formation of the lesions. The lesions generally persist for 10 to 14 days, although, in some cases they may last longer, especially in immunocompromised patients. A further consequence of the lesions, aside from cosmetic unsightliness and serious physical discomfort, is that they are a potential avenue for spreading the virus either to other locations on the body or to other individuals, i.e., lesions are a major avenue of further contamination. It has remained desired to have a means of minimizing the severity and duration of lesions caused by herpetic infections.

For most viral infections, treatment is difficult because antiviral agents are often toxic to varying degrees and/or cause undesirable side effects to the subject, particularly when administered systemically. Therefore, the use of a topical composition to combat the viral infection is particularly useful. As a means of treatment, a topical composition offers a number of advantages, such as, minimizing the compromised subject's systemic exposure to the antiviral agent and providing a means of rapidly delivering the antiviral agent in high concentrations to the active site of viral multiplication.

Accordingly, it would be advantageous to have a composition that is effective in the topical treatment of viral infections and which contains a minimum amount of a potent antiviral agent. Such a combination would avoid or minimize the toxic side effects often associated with antiviral agents.

Surfactants are a useful means of treating viral infections because many do not present a toxicity problem. Nonionic surfactants, in particular, have been found to be useful in the treatment of viral infections as discussed in U.S. Patent No. 4,020,183, incorporated herein by reference.

European Patent Application No. 77,063 discloses compositions containing antiviral surfactants and interferon. [See also, Antimicrob. Agents Chemother., 13(4), 686-90 (1978) and Antimicrob. Agents Chemother., 28(3), 449-51 (1985)]. While nonionic surfactants have been shown to exhibit antiviral activity in in vitro testing, such antiviral activity has not been consistently demonstrated in vivo. [(See, Am. J. Obstet. Gynecol., 133, 548 (1979)].

Azone and nonoxynol-9 are both nonionic surfactants, however, they belong to different genuses within the larger families. Structurally, they can be distinguished by the chemical linkage between the hydrophilic and lipophilic moieties of the compounds, i.e., amide linkage for Azone and ether linkage for Nonoxynol-9. Also, their hydrophilic moieties are quite distinct; Azone has a lactam functionality; and Nonoxynol-9 has a nine monomer polyoxyethylene chain.

The modes of action reported for each compound are likewise distinct. Azone has been reported as a penetration enhancer, i.e., when it is co-administered with another agent, Azone will facilitate the movement of the agent into the cell by increasing lipid fluidity (Lambert, et al., (1989), Pharm Res., 6(9):798-803). The enhancement effect has been reported to be more pronounced with lipophilic agents and with agents with higher partition coefficients. A partition coefficient is the ratio of the concentrations of a given compound in a nonpolar medium, e.g., octanol and in an aqueous medium, e.g., water (ACV and DHPG have partition coefficients of less than 0.1, whereas Ada-DHPG has a partition coefficient of about $2.8 \times 10^5$). It has also been reported that Azone is most effective as a penetration enhancer when used with a polar cosolvent, e.g., propylene glycol (Wotton et al., (1985), Int. J. Pharm., 24:19-26).

In antiviral therapies, Azone has been reported to improve the efficacy of topically applied antiviral compounds against cutaneous herpes virus by improving skin permeability of the antiviral compound (Spruance et al., (1984), Antimicrobial Agents Chemother., 26(6):819-23; Kumar et al., (1987), Curr. Ther. Res., 41(6) :02-08; and Shannon et al., (1985), J. Pharm. Sci., 74(11):1157-61). Specifically, a combination of Azone and Acyclovir has been reported to be effective in treating herpes simplex infections using an immunocompromised baby guinea pig model (Current Therapeutic Research, 41, 6, 802, 1987).

Nonoxynol-9 is known primarily as a spermicide and viricide. It has been reported to effective again herpes simplex virus (HSV) and human immunodeficiency virus (HIV). Nonoxynol-9 when administered with human leukocyte interferon has been reported to exhibit a synergistic effect in the treatment of herpes simplex virus type 2 (Rapp and Wrzos, (1985), Antimicrobial Agents and Chemother., 28(3):449-51). Nonoxynol-9 has been reported to act against the herpes virus by dissolving the viral envelope and partially degrading the nucleocapsid (Zehnder and Hart, (1989), DICP, The Annals of Pharmacotherapy, 23:602). It has surprisingly, been discovered that certain pharmaceutically acceptable surfactants greatly enhance the in vivo activity of antiviral agents, such as, 9-[1,3-di-(1-adamantylcarboxy)-2-propoxymethyl]guanine (or Ada-DHPG), 9-(1,3-dihydroxy-2-propoxymethyl)guanine (or "DHPG"), and 9-(2-hydroxyethoxymethyl)guanine (or "Acyclovir"). This unexpected enhancement of activity facilitates excellent topical treatment of viral infections using minimal levels of antiviral agents.

Accordingly, it is an object of the present invention to provide novel pharmaceutical compositions containing antiviral agents and nonionic surfactants, useful for the topical treatment of viral infections.

It is a further object of the present invention to provide a method for treating lesions due to viral infections by topically administering the aforesaid pharmaceutical composition.

It is a further object of the present invention to provide an improved process for manufacturing the aforesaid pharmaceutical topical compositions, to increase shelf life by delaying formulation separation into component ingredients.

This invention relates to a composition useful for topically treating viral infections in mammals, the composition including a therapeutically effective amount of an active antiviral agent, a pharmaceutically acceptable nonionic surfactant in a quantity sufficient to enhance the antiviral activity of said composition, and optionally pharmaceutically acceptable excipients in quantity sufficient to make weight.

This invention also relates to a method of topically treating viral infections in mammals, by applying the above described antiviral composition to the site of infection.

This invention also relates to a process for manufacturing the aforesaid pharmaceutical composition.

Definitions

Unless otherwise specified, the following terms as used in the Specification and appended Claims have the meaning indicated below.

"Pharmaceutically acceptable" refers to the following characteristics in components of the composition:

    i) they will not have an untoward or deleterious effect on the host to which they are administered,
    ii) they will not cause unacceptable irritation to mucosal or skin membranes, and/or
    iii) they will not cause toxicity when used in the normal and usual course of drug administration.

"Quantity sufficient" or "q.s." means adding a quantity sufficient to achieve a stated function, e.g., to bring a solution to a desired volume.

"Treatment" or "treating" refers to any means of treatment of a disease in a mammal, including:

    (i) preventing the disease, that is, causing the clinical symptoms of the disease not to develop;
    (ii) inhibiting the disease, that is, arresting the development of clinical symptoms; and/or
    (iii) relieving the disease, that is, causing the regression of clinical symptoms.

"Effective amount" is a dosage sufficient to provide treatment for the disease state being treated. This will vary depending on the patient, the disease and the treatment being affected.

"Surfactant" or "surface-active agent" is a compound which lowers the surface tension of the medium in which it is dissolved. Structurally, surfactants are amphipathic, i.e., they possess both non-polar (e.g. hydrophobic) and polar (e.g. hydrophilic) moieties within the same molecule.

A "nonionic surfactant" is a surfactant that does not carry a charge. Nonionic surfactants can be classified according to the type of functional group linking the hydrophilic and hydrophobic portions of the molecule. The nonionic surfactants useful in the present invention have at least one ether linkage between the hydrophilic and hydrophobic portions of the molecule.

3

## Formulations

The present invention may be embodied in compositions of the following forms; cream, gel, lotion, oil, emulsion, ointment, foam, aerosol, solution and the like.

The formulations of the present invention include an antiviral agent in an effective amount for topical treatment of viral infections, one or more nonionic surfactants in a quantity sufficient to enhance the activity of the antiviral agent, and optionally including other pharmaceutically acceptable excipients, such as, adjuvants, gelling agents, stabilizers, antioxidants, emollients, emulsifiers, preservatives, solvents and the like. The ingredients used in the formulations of the present invention are typically commercially available or can be made by one skilled in the art.

The invention relates primarily to topical formulations of an antiviral active agent and a nonionic surfactant, which components, when combined in the composition, act synergistically exhibiting far greater antiviral activity than would be expected from combining the activities of the individual components.

## The Active Agents

The antiviral active agents useful in the present invention include, e.g., 9-[1,3-di-(1-adamantylcarboxy)-2-propoxy-methyl]guanine (or Ada-DHPG), 9-(1,3-dihydroxy-2-propoxymethyl)guanine (or "DHPG"), and 9-(2-hydroxyethoxymethyl)guanine (or "Acyclovir"), which are prepared and described, for example, in U.S. Patents Nos. 4,612,314; 4,355,032; and 4,199,574, respectively, all incorporated herein by reference.

An effective amount of antiviral active agent (such as 9-[1,3-di-(1-adamantylcarboxy)-2-propoxymethyl]-guanine) in the compositions of the present invention may be between about 0.01% and 30% weight/weight (w/w) with the preferred range being about 1% to 10% w/w.

## The Surfactants

The nonionic surfactants that are useful in the present invention are compounds which enhance the activity of the antiviral agent, including those having at least one ether linkage between the hydrophilic and hydrophobic portions of the molecule. Examples of this type of surfactant include polyoxyethylene alkyl-phenols, polyoxyethylene alcohols, polyoxyethylene esters of fatty acids, polyoxyethylene mercaptans polyoxyethylene alkyl amines, nonyl-phenoxy-polyethoxyethanol (or "Nonoxynol-9", commercially available from Whitehall Laboratories, Inc. Division of American Home Products Corporation, 685 Third Ave, New York, N.Y. 10017), and the like.

An effective amount of the surfactant in the present invention may be from about 1% to about 99.99995% by weight, with a preferred range about 1 to 95% For example, in a cream formulation the surfactant comprises about 1% to 15% by weight with the preferred range being about 1% to 10% by weight. In a gel formulation, the effective amount of the surfactant comprises about 1% to 99.99995% by weight with the preferred range being about 5% to 95% by weight.

The surfactant may act as a vehicle for gel formulation as well as an enhancer for the antiviral agent, hence the overall amount of surfactant in the formulation may be accordingly increased.

## Preparation

The compositions are prepared by methods known to one skilled in the formulation art and may contain excipients which are also known in the art, such as adjuvants, gelling agents, stabilizers such as antioxidants and the like. A generally recognized compendium of such methods and ingredients is Remington's Pharmaceutical Sciences edited by Arthur Osol, (Mack Printing Co., 17th Edition, 1985).

The formulations of these compositions are prepared by combining an antiviral active agent and one or more surfactants in a pharmaceutically acceptable vehicle. The vehicle may be the surfactant itself or any suitable pharmaceutically acceptable vehicle such as polyethylene glycol, mineral oil, petrolatum, propylene glycol, glycerin, water and the like. The vehicle may also be a mixture of the surfactant with another suitable solvent or a mixture of various suitable solvents.

Formulations, of the present invention as a gel composition, are prepared, e.g., from the listed ingredients in the following approximate ranges.

| Ingredient | % W/W |
|---|---|
| Antiviral agent | 1 to 10 |
| Carbopol 940 | 1.25 to 2.5 |
| 0.1N Sodium hydroxide | 2.5 to 5 |
| Butylated hydroxytoluene | 0.005 to 0.01 |
| Surfactant | q.s. to 100% |

To prepare the gel composition, the antiviral agent and butylated hydroxytoluene are dissolved in the surfactant (e.g., Nonoxynol-9) with mild heating. Carbopol 940, a gelling agent, is added and gently stirred until dissolved. Sodium hydroxide is then added. The resultant liquid is cooled to room temperature to form a gel.

Formulations, of the present invention as a cream composition with the surfactant (e.g., Nonoxynol-9), are prepared, e.g., from the listed ingredients in the following approximate ranges.

| Ingredient | % W/W |
|---|---|
| Antiviral Agent | 1 to 10 |
| Mineral Oil | 1 to 10 |
| Stearyl Alcohol | 1 to 10 |
| Cetyl Alcohol | 2 to 6 |
| Tween 60 (Polysorbate 60) | 2 to 4 |
| Span 60 (Sorbitan Monostearate) | 1 to 2 |
| Surfactant | 1 to 20 |
| Methyl Paraben | 0.09 to 0.18 |
| Propyl Paraben | 0.02 to 0.05 |
| Butylated hydroxytoluene | 0.005 to 0.01 |
| Dimethicone | 0.005 to 0.01 |
| Glycerin | 5 to 15 |
| Water | q.s. to 100% |

To prepare the cream composition, all of the ingredients listed for the cream composition (except water, glycerin and the antiviral agent) are combined and heated at 65 to 80° C with stirring. A slurry of the antiviral agent is prepared with a small amount of a water/glycerin mixture. The remaining quantity of water and glycerin at 65 to 80° C is then added to the above ingredient mixture with vigorous stirring. The cream is cooled to room temperature and the slurry containing the antiviral agent is then added with stirring. The preferred range for Nonoxynol-9 is 1 to 7% w/w.)

By manufacturing the formulations of the present invention according to the above described process, it is possible to avoid problems arising from the use of more customary manufacturing processes where such formulations have shown a tendency to separate into their components, thus decreasing their stability. Formulations made by the process of the instant invention do not present this problem, and therefore benefit from increased stability and shelf life.

Preferred Formulations

Although pharmaceutically acceptable anionic, cationic or ampholytic surfactants maybe useful in the present invention, pharmaceutically acceptable nonionic surfactants are preferred.

Preferred are nonionic surfactants having at least one ether linkage between the hydrophobic and hydrophilic portions of the molecule. Most preferred is nonylphenoxy-polyethoxyethanol (Nonoxynol-9).

Particularly preferred are formulations including 9-[1,3-di-(1-adamantylcarboxy)-2-propoxy-methyl]-guanine and Nonoxynol-9.

Also particularly preferred are formulations including 9-(2-hydroxyethoxymethyl)guanine and Nonoxynol-9.

Utility, Testing and Administration

The compositions of the present invention are useful for treating any external manifestations of a viral infection, in particular, infections caused by Herpes Simplex 1, Herpes Simplex 2 and vericella Zoster virus.

Topical administration of the instant composition may be effected by applying a small amount of the composition directly to and in areas adjacent to the site of the lesions with the fingers (while wearing protective gloves), a cotton swab, soft brush, sponge or the like. A quantity sufficient to cover the area of the lesions is effective. Treatment with the pharmaceutical compositions may be once a day or as frequent, for example, as every 2 to 4 hours. Whatever the frequency of application the treatment should be continued for about 3 to 6 days. Preferably, the pharmaceutical compositions are applied during an early stage of the viral infection.

In vivo testing for antiviral activity against cutaneous HSV-1 infections is done using a mouse model, for example, by the procedures described by Lieberman, M., Schafer, T.W., and Came, P., "Chemotherapy of Cutaneous Herpesvirus Infection of Hairless Mice", J. Invest. Dermatol., 60:203-206 (1973).

In vivo testing for antiviral activity against cutaneous HSV-1 infections is also done using a guinea pig model, for example, by the procedures described by Hubler, W.R., Jr., Troy, D.F., Troll, D., and Jarratt, M., "Guinea Pig Model For Cutaneous Herpes Simplex Virus Infection", J. Invest. Dermatol., 62:92-95 (1974).

In vivo testing for antiviral activity against genital HSV-2 infections is done using a mouse model, for example, by the procedures described by Kern, E.R., "Acyclovir Treatment of Experimental Genital Herpes Simplex Virus Infections. Symposium on Acyclovir", Amer. J. Med., 73(1a):100-108 (1982).

The following examples are given to enable those skilled in the art to more clearly understand and to practice the present invention. They should not be considered as a limitation on the scope of the invention, but merely as being illustrative and representative thereof.

## EXAMPLE 1

This example illustrates the preparation of a representative pharmaceutical formulation for topical administration containing an antiviral active agent, e.g., 9-[1,3-di(1-adamantylcarboxy)-2-propoxymethyl]-guanine.

| 10% Hydrous Cream Formulation | |
|---|---|
| Ingredient | % W/W |
| Antiviral agent | 10.00 |
| Mineral Oil # 35, USP | 6.00 |
| Stearyl Alcohol, NF | 6.00 |
| Cetyl Alcohol, NF | 4.00 |
| White Wax, NF | 5.00 |
| Polysorbate 60, NF | 2.00 |
| Sorbitan monostearate, NF | 2.00 |
| Nonoxynol-9, USP | 5.00 |
| Methylparaben, NF | 0.18 |
| Propylparaben, NF | 0.05 |
| Benzyl Alcohol, NF | 1.00 |
| Butylated Hydroxytoluene, NF | 0.01 |
| Dimethicone, NF | 0.01 |
| Glycerin, USP | 5.00 |
| Purified Water, USP q.s. | 100.00 |

## MANUFACTURING PROCEDURE

1. To an appropriate mixer, add mineral oil # 35, stearyl alcohol, cetyl alcohol, white wax, polysorbate 60, sorbitan monostearate, methyl paraben, propyl paraben, benzyl alcohol, butylated hydroxytoluene and dimethicone.

2. Heat liquid (1) to 70 to 80° C and stir until it is thoroughly mixed.

3. In a separate container, heat the mixture of glycerin and purified water to 70 to 80° C.

4. Add (3) to (2) while mixing with a homomixer set at 3,600 RPM.

5. Homomix for 10 minutes (under vacuum -20 psig).

6. Reduce homomixer speed to 1800 RPM and start cooling.

7. Stop homomixing when the cream base congeals.

8. Continue to mix the cream base with a planetary paddle.

9. When the cream base's temperature reaches 25° C, add Nonoxynol-9 into (8) in small portions.

10. After all the Nonoxynol-9 is added, continue mixing for 30 minutes.

11. Transfer a known weight of the cream base to a clean flat surface; levigate appropriate amount of active antiviral agent into the cream base homogenously.

## EXAMPLE 2

| Cream Formulation with Nonoxynol-9 | |
|---|---|
| Ingredient | % W/W |
| Antiviral agent | 10.00 |
| Mineral Oil # 35, USP | 6.00 |
| Stearyl Alcohol, NF | 6.00 |
| Cetyl Alcohol, NF | 4.00 |
| White Wax, NF | 5.00 |
| Polysorbate 60, NF | 2.00 |
| Sorbitan monostearate, NF | 2.00 |
| Nonoxynol-9, USP | 5.00 |
| Methylparaben, NF | 0.18 |
| Propylparaben, NF | 0.05 |
| Benzyl Alcohol, NF | 1.00 |
| Butylated Hydroxytoluene, NF | 0.01 |
| Dimethicone, NF | 0.01 |
| Glycerin, USP | 5.00 |
| Purified Water, USP q.s. | 100.00 |

All of the above ingredients, except water, glycerin, Nonoxynol-9 and the antiviral agent, are combined and heated at 80° C with stirring. The antiviral agent is slurried in a small amount of a water/glycerin/Nonoxynol-9 mixture. The remaining quantity of water and glycerin at 65 to 80° C is then added to the above ingredient mixture with vigorous stirring. The cream is cooled to room temperature and the slurry containing the antiviral agent is then added with stirring.

## EXAMPLE 3

| Gel Formulation with Nonoxynol-9 | |
| --- | --- |
| Ingredient | % W/W |
| Antiviral agent | 1 |
| PG 8000 | 5 |
| Stearyl Alcohol | 10 |
| Butylated hydroxytoluene | 0.01 |
| Nonoxynol-9 | q.s. to 100% |

All of the ingredients are warmed to 70°C with mixing. The resultant liquid is then cooled to room temperature to form the gel.

EXAMPLE 4

| Cream Formulation with Azone | |
| --- | --- |
| Ingredient | % W/W |
| Antiviral agent | 10.00 |
| Mineral Oil # 35, USP | 6.00 |
| Stearyl Alcohol, NF | 6.00 |
| Cetyl Alcohol, NF | 4.00 |
| White Wax, NF | 5.00 |
| Polysorbate 60, NF | 2.00 |
| Sorbitan monostearate, NF | 2.00 |
| Azone, USP | 10.00 |
| Methylparaben, NF | 0.18 |
| Propylparaben, NF | 0.05 |
| Benzyl Alcohol, NF | 1.00 |
| Butylated Hydroxytoluene, NF | 0.01 |
| Dimethicone, NF | 0.01 |
| Glycerin, USP | 5.00 |
| Purified Water, USP q.s. | 100.00 |

All of the above ingredients, except water, glycerin, Azone and the antiviral agent, are combined and heated at 80°C with stirring. The antiviral agent is slurried in a small amount of a water/glycerin/Nonoxynol-9 mixture. The remaining quantity of water and glycerin at 65-80°C is then added to the above ingredient mixture with vigorous stirring. The cream is cooled to room temperature and the slurry containing the antiviral agent is then added with stirring.

EXAMPLE 5

Efficacy of 9-[1,3-di(1-adamantylcarboxy)-2-propoxymethyl]guanine In Combination With Propylene Glycol and Nonoxynol-9 Applied Topically Against Cutaneous HSV-1 in Guinea Pig

This procedure is a modification of a procedure initially described by Hubler, W.R., Jr., et al., "Guinea Pig Model For Cutaneous Herpes Simplex Virus Infection", J. Invest. Dermatol., 62:92-95 (1974).

The backs of female Hartley guinea pigs were shaved, depilated and inoculated with HSV-1 (F strain) in four different areas by multiple shallow punctures. Two quadrants were on the left side and two on the right side of the dorsum. The animals were divided into 4 groups of 6 animals each and topically administered a cream on one side of the back (0.5 ml/animal) once a day for 3 days starting 24 hour post-infection. The other side of the back was not treated. Two of the groups were administered placebo creams which contained all of the ingredients except Ada-DHPG, i.e., either a 5% Nonoxynol-9 (N-9)-based cream or a, 5% propylene glycol (PG)-based cream. A third group was treated with a cream containing a formulation of 5% Nonoxynol-9 (N-9) and 10% 9-[1,3-di(1-adamantylcarboxy)-2-propoxy-methyl]guanine (Ada-DHPG). The fourth group was not treated on either side of the back with any cream. The number of lesions which developed were counted on Day 4 post-infection. The treated side versus the nontreated side for the various groups were compared by a percent reduction in the number of lesions which developed. The results are given below in Table 1.

TABLE 1

| Treatment | Percent Reduction |
|---|---|
| untreated on either side | 26 |
| 5% PG | 37 |
| N-9 (only) | 31 |
| Ada-DHPG/N-9 | 93* |

\* P value less than 0.05 comparing treated side with untreated side using Mann Whitney U test

EXAMPLE 6

Efficacy of 9-[1,3-di(1-adamantylcarboxy)-2-propoxy-methyl]guanine In Combination With Nonoxynol-9 Applied Topically Against Cutaneous HSV-1 Infection in Mouse

This is a modification of the procedure initially described by Lieberman, M., Schafer, T.W., and Came, P., "Chemotherapy of Cutaneous Herpesvirus Infection of Hairless Mice", J. Invest. Dematol., 60:203-206 (1973).

The backs of female hairless HRS hr hr strain mice were lightly abraded and rubbed with HSV-1 (F strain). The mice were then divided into 3 groups of 20 mice each. Each mouse was topically administered a cream on the back (0.05 ml/mouse) once a day for 5 days starting 24 hour post-infection. Two groups were treated with a cream containing 5% Nonoxynol-9 (N-9) or alternatively a formulation of 5% Nonoxynol-9 (N-9) and 10% 9-[1,3-di(1-adamantylcarboxy)-2-propoxymethyl]guanine (Ada-DHPG). The third group was not administered treatment and served as a control group. The developing lesions were scored for severity on a 0-4 scale, with a score of 4 being the most severe, on days 1 to 14 post-infection. The treated versus nontreated scores for the various groups were compared by a percent reduction in the overall mean severity score of the lesions which developed.

The results are reported below in Table 2.

TABLE 2

| Treatment | Percent Reduction |
|---|---|
| N-9 (only) | 9 |
| Ada-DHPG/N-9 | 86* |

* P value less than 0.05 comparing treated groups with the untreated group using Mann Whitney U test

## EXAMPLE 7

Efficacy of 9-[1,3-di(1-adamantylcarboxy)-2-propoxy-methyl]guanine In Combination With Nonoxynol-9 Applied Topically Against Vaginal HSV-2 Infection In Mouse

This is a modification of the procedure initially described in Kern, E.R., "Acyclovir Treatment of Experimental Genital Herpes Simplex Virus Infections. Symposium on Acyclovir." Amer J Med. 73(1a):100-108 (1982).

Female Swiss Webster mice were vaginally infected with HSV-2 (G strain) and divided into three groups of 20 mice each for treatment. One group was untreated and served as the control. The other groups were topically administered a cream containing either 5% Nonoxynol-9 (N-9) or alternatively a formulation of 5% Nonoxynol-9 (N-9) and 10% 9-[1,3-di-(1-adamantylcarboxy)-2-propoxy-methyl]guanine (Ada-DHPG). The creams were applied intravaginally (0.05ml/animal) once a day for five days starting 24 hour post-infection. The developing vaginal lesions were scored for severity on a 0 to 4 scale, with a score of 4 being the most severe, on days 4 to 15 post-infection. The treated versus nontreated scores for the various groups were compared by a percent reduction in the overall mean severity score of the lesions which developed. The results are given below in Table 3.

TABLE 3

| Treatment | Percent Reduction |
|---|---|
| Nonoxynol-9 (only) | 0 |
| Ada-DHPG/N-9 | 87* |

* P value less than 0.05 comparing treated groups with the untreated group using Mann Whitney U test

## EXAMPLE 8

Efficacy of 9-[1,3-di(1-adamantylcarboxy)-2-propoxymethyl]guanine In Combination With Azone and Nonoxynol-9 Applied Against HSV-1 Cutaneous Infection in Guinea Pigs

This procedure is a modification of a procedure initially described by Hubler, W.R., Jr., et al., "Guinea Pig Model For Cutaneous Herpes Simplex Virus Infection", J. Invest. Dermatol., 62:92-95 (1974).

The backs of female Hartley guinea pigs were shaved, depilated and inoculated with HSV-1 (F strain) in four different areas by multiple shallow punctures. Two quadrants were on the left side and two on the right side of the dorsum. The guinea pigs were divided into treatment groups of usually 6 each. Treatment was topically administered once a day for 3 days starting 24 hours post-infection on one side of the back. The other side of the back was not treated. Different treatment groups were administered creams which contained 9-[1,3-di(1-adamantyl-carboxy)-2-propoxymethyl]guanine (Ada-DHPG), (10%, 5%, 2.5%, 1% or 0%) with 5% propylene glycol (PG), or 5% propylene glycol (PG); and either Nonoxynol-9 (5% and 2.5%) or Azone (10% and 5%). Additionally, other groups were administered creams containing Ada-DHPG (10%, 5%, 2.5%, 1% or 0%) with no propylene glycol and either 10% Azone or 5% Nonoxynol-9. Seven dose response tests were run in all. In each test the numbers of lesions were counted on Day 4 after infection. The treated side versus the nontreated side for the various groups were compared by a percent reduction in number of lesions which developed. The results are compiled in Table 4.

TABLE 4

| Test No. | Formulation | Concentration of Ada-DHPG | Percent Reduction |
|---|---|---|---|
| 1 | 5% PG (no surfactant added) | | |
| | | 10 | 37* |
| | | 5 | 3 |
| | | 2.5 | 16 |
| | | 1 | 12 |
| | | 0 (placebo) | 13 |
| 2 | 5% Azone | | |
| | | 10 | 29 |
| | | 5 | 14 |
| | | 2.5 | 14 |
| | | 1 | -6 |
| | | 0 (placebo) | 10 |
| 3 | 10% Azone | | |
| | | 10 | 78* |
| | | 5 | 73* |
| | | 2.5 | 35* |
| | | 1 | 43* |
| | | 0 (placebo) | 27 |
| 4 | 10% Azone (no PG) | | |
| | | 10 | 47* |
| | | 5 | 27 |
| | | 2.5 | 18 |
| | | 1 | 27 |
| | | 0 (placebo) | 35 |
| 5 | 2.5% Nonoxynol-9 | | |
| | | 10 | 64* |
| | | 5 | 52* |
| | | 2.5 | 52* |
| | | 1 | 33 |
| | | 0 (placebo) | 26 |
| 6 | 5% Nonoxynol-9 | | |
| | | 10 | 88* |
| | | 5 | 92* |
| | | 2.5 | 65* |
| | | 1 | 59* |
| | | 0 (placebo) | 38 |
| 7 | 5% Nonoxynol-9 (no PG) | | |
| | | 10 | 82* |
| | | 5 | 90* |
| | | 2.5 | 68* |
| | | 1 | 57* |
| | | 0 (placebo) | 37 |

* P value less than 0.05 comparing treated side with untreated side using Mann Whitney U test.

EXAMPLE 9

Efficacy of Acyclovir In Combination With Nonoxynol-9 Applied Topically Against Cutaneous HSV-1 Infections in Guinea Pigs

This procedure is a modification of a procedure initially described by Hubler, W.R., Jr., et al., "Guinea Pig Model Eor Cutaneous Herpes Simplex Virus Infection", J. Invest. Dermatol., 62:92-95 (1974).

The backs of female Hartley guinea pigs were shaved, depilated and inoculated with HSV-1 (F strain) in four different areas by multiple shallow punctures. Two quadrants were on the left side and two on the right side of the dorsum. The animals were divided into 3 treatment groups of 6 animals each. The treatment groups were topically administered a cream on one side of the back once a day for 3 days starting 24 hours post-infection, the other side of back was not treated. The creams contained as an active ingredient either 5% acyclovir in a polyethylene glycol (PEG) based cream (Zovirax ®), 5% acyclovir in a 5% Nonoxynol-9 (N-9) cream or 5% Nonoxynol-9 cream only (placebo control). The numbers of lesions which developed on either the treated or untreated side of the back were counted on Day 4 after infection. The treated side versus the nontreated side for the various groups were compared by a percent reduction in numbers of lesions which developed. The results are given below in Table 5.

TABLE 5

| Formulation | Percent Reduction |
|---|---|
| 5% ACV in PG based commercial cream (Zovirax ®) | 6 |
| 5% ACV in 5% Nonoxynol-9 cream | 69* |
| 5% Nonoxynol-9 (placebo) | 30 |

* P value less than 0.05 comparing treated side with untreated side using Mann Whitney U

EXAMPLE 10

Efficacy of 9-(1,3-dihydroxy-2-propoxymethyl)guanine and 9-[1,3-di(1-adamantylcarboxy)-2-propoxymethyl]-guanine In Combination With Azone, Nonoxynol-9, and Propylene Glycol Applied Topically Against Cutaneous HSV-1 Infections in Guinea Pigs

This procedure is a modification of a procedure initially described by Hubler, W.R., Jr., et al., "Guinea Pig Model For Cutaneous Herpes Simplex Virus Infection", J. Invest. Dermatol., 62:92-95 (1974).

The backs of female Hartley guinea pigs were shaved, depilated and inoculated with HSV-1 (F strain) in four different areas by multiple shallow punctures. Two quadrants were on the left side and two on the right side of the dorsum. The animals were divided into groups and topically administered a cream on one side of the back (0.5 ml/animal) once a day for 3 days starting 4 days post infection. The other side of the back was not treated. The cream contained either a formulation of 9-(1,3-dihydroxy-2-propoxymethyl)guanine (DHPG) with Azone, Nonoxynol-9 (N-9), or propylene glycol (PG); or 9-[1,3-di(1-adamantylcarboxy)-2-propoxymethyl]guanine (Ada-DHPG) with Azone, Nonoxynol-9 (N-9), or propylene glycol (PG). Placebo groups were treated with only Azone, Nonoxynol-9 (N-9) or propylene glycol (PG). Both DHPG and Ada-DHPG were run in each test with a single formulation. A total of 4 tests were run. In each study, the number of lesions which developed were counted on Day 4 after infection. The treated side versus nontreated side for the various groups were compared by a percent reduction in the number of lesions which developed. The results are given below in Table 6.

TABLE 6

| Test No. | Formulation | Treatment | Percent Reduction |
|---|---|---|---|
| 1 | 25% PG | | |
| | | 10% Ada-DHPG** | 51* |
| | | 4.4% DHPG | 75* |
| | | 25% PG only | 37 |
| 2 | 5% PG | | |
| | | 10% Ada-DHPG** | 42* |
| | | 4.4% DHPG | 68* |
| | | 5% PG only | 20 |
| 3 | 10% Azone | | |
| | | 10% Ada-DHPG** | 76* |
| | | 4.4% DHPG | 100* |
| | | Azone only | 40 |
| 4 | 5% Nonoxynol-9 | | |
| | | 10% Ada-DHPG** | 77* |
| | | 4.4% DHPG | 94* |
| | | 5% N-9 | 38 |

\* P value less than 0.05 comparing treated side with untreated side using Mann Whitney U

\*\* 10% Ada-DHPG is equimolar with 4.4% DHPG

**Claims**

1. A pharmaceutical composition useful for topical treatment of viral infections in mammals, said composition comprising a therapeutically effective amount of antiviral active agent, a pharmaceutically acceptable nonionic surfactant, wherein said surfactant is one having at least one ether linkage between the hydrophobic and hydrophilic portions of the molecule, in quantity sufficient to enhance the antiviral activity of said composition, and optionally, pharmaceutically acceptable excipients in quantity sufficient to make weight.

2. The pharmaceutical composition of Claim 1, wherein said surfactant is nonyl-phenoxy-polyethoxyethanol.

3. The pharmaceutical composition of Claim 1 or 2, wherein said composition is a gel.

4. The pharmaceutical composition of Claim 3, wherein said surfactant is present at an amount from 5% to 95% by weight.

5. The pharmaceutical composition of Claim 1 or 2, wherein said composition is a cream.

6. The pharmaceutical composition of Claim 5, wherein the amount of nonionic surfactant is from 1% to 15% by weight.

6. The pharmaceutical composition of Claim 5, wherein the amount of nonionic surfactant is from 1% to 15% by weight.

7. The pharmaceutical composition of any one of Claims 1-6, wherein the antiviral active agent is:
9-[1,3-di-(1-adamantylcarboxy)-2-propoxymethyl]-guanine,
9-(1,3-dihydroxy-2-propoxymethyl)guanine, or
9-(2-hydroxyethoxymethyl) guanine.

8. The pharmaceutical composition of Claim 7, wherein said composition comprises 0.01% to 30% w/w of 9-(2-hydroxyethoxymethyl)guanine; 1% to 99% w/w of a pharmaceutically acceptable nonionic surfactant; and pharmaceutically acceptable excipients in quantity sufficient to make weight.

9. The pharmaceutical composition of Claim 8, having from 0.01% to 10% w/w of 9-(2-hydroxymethoxyethyl)guanine.

10. The pharmaceutical composition of any one of Claims 6-9, wherein the amount of nonionic surfactant is from 1% to 7% by weight.

11. The pharmaceutical composition of any one of claims 1-10 useful for topical treatment of herpes simplex 1 or herpes simplex 2 virus infections.

12. A process of manufacturing a cream composition according to any one of Claims 5-11 which process comprises the steps of:

a. heating a mixture of excipients to elevated temperature, stirring to uniformity, and cooling until the mixture begins to congeal;

b. adding surfactant into said congealing creambase in small portions, at about room temperature; and

c. levigating an antiviral active agent into the creambase/surfactant mixture.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | "Rote Liste", 1986, Editio Cantor, Aulendorf/Württemberg, DE<br>* No. 31 081, "Zovirax" * | | A 61 K 31/765 //<br>(A 61 K 31/765<br>A 61 K 31:52 ) |
| A,D | US-A-4 020 183 (SAMUEL SIMON ASCULAI) | 1-12 | |
| A,D | EP-A-0 077 063 (VIRAL GENETICS, INC.) | 1-12 | |

TECHNICAL FIELDS SEARCHED (Int. Cl.5)

A 61 K

The present search report has been drawn up for all claims

| Place of search | Date of completion of the search | Examiner |
|---|---|---|
| THE HAGUE | 30-07-1990 | BRINKMANN C. |

EPO FORM 1503 03.82 (P0401)